# EUROPEAN PATENT SPECIFICATION

(11) **EP 3 305 202 B1**
(45) Date of publication and mention of the grant of the patent: **06.03.2024**
(21) Application number: 17194989.4
(22) Date of filing: 05.10.2017
(51) Int. Cl.: A61B 8/08, A61B 6/00, A61B 8/00, A61B 8/12, A61B 34/20, A61B 6/12

(54) **PRE-OPERATIVE REGISTRATION OF ANATOMICAL IMAGES WITH A POSITION-TRACKING SYSTEM USING ULTRASOUND**
PRÄOPERATIVE REGISTRIERUNG ANATOMISCHER BILDER MIT EINEM POSITIONSVERFOLGUNGSSYSTEM MIT ULTRASCHALL
ENREGISTREMENT PRÉ-OPÉRATOIRE D'IMAGES ANATOMIQUES COMPRENANT UN SYSTÈME DE DÉTERMINATION DE POSITION À L'AIDE D'ULTRASONS

(30) Priority: 06.10.2016 US 201615286891
(43) Date of publication of application: 11.04.2018
(73) Proprietor: Biosense Webster (Israel) Ltd., Yokneam, 2066717 (IL)
(72) Inventor: GOVARI, Assaf, 2066717 Yokneam (IL); GLINER, Vadim, 2066717 Yokneam (IL)
(74) Representative: Carpmaels & Ransford LLP

(56) References cited:
- WO-A1-2006/092602
- DE-A1-102012 103 725
- US-A- 6 006 126
- US-A1- 2002 035 321
- US-A1- 2004 236 223
- US-A1- 2014 276 001
- US-A1- 2016 008 083
- DIAKOV G ET AL: "Automatische Registration des Patienten mit A-Mode-Ultraschall fÃ 1/4 r computerunterstÃ 1/4 tzte Chirurgie ; Funktionsnachweis im Labor ; Automatic registration of patients with A-mode ultrasound for computer-assisted surgery ; Laboratory proof of concept", HNO ; DEUTSCHE GESELLSCHAFT FÜR HALS-NASEN-OHREN-HEILKUNDE, KOPFUND HALS-CHIRURGIE, SPRINGER, BERLIN, DE, vol. 58, no. 11, 30 September 2010 (2010-09-30), pages 1067-1073, XP019858654, ISSN: 1433-0458, DOI: 10.1007/S00106-010-2171-1

## Description

### FIELD OF THE INVENTION

The present invention relates generally to image guided medical procedures, and particularly to systems for registration of an anatomical image with a position-tracking system.

### BACKGROUND OF THE INVENTION

Ultrasound (US) transducers and position-tracking systems may be used in various medical applications, such as image guided procedures.

For example, U.S. Patent 5,575,288 describes an ultrasonic imaging system having a remote ultrasound console and a probe connected thereto for inspecting an interior region of a body. The ultrasonic imaging system includes a scan-head housing disposed at a distal end of the probe. A transducer is mounted upon a support structure within the scan-head housing and is electrically connected to the ultrasonic imaging system. A magnetic position sensor is located within the scan-head housing and coupled to the ultrasonic imaging system.

U.S. Patent 7,751,868 describes an integrated skin-mountable multifunction device for use with a computer assisted or image guided surgical system and methods of using the same. The multifunction device includes a patient mountable portion that has at least one position indicating element and at least one imageable pattern whose geometry is known in a coordinate system of the at least one position indicating element, wherein the imageable pattern is visible on an imaging modality.

U.S. Patent Application Publication 2006/0253031 describes a system and method for imaging a target in a patient's body uses a pre-acquired image of the target and a catheter having a position sensor and an ultrasonic imaging sensor. The catheter is placed in the patient's body and positional information of a portion of the catheter in the patient's body is determined using the position sensor. The catheter is used to generate an ultrasonic image of the target using the ultrasonic imaging sensor. An image processor is used for determining positional information for any pixel of the ultrasonic image of the target and registering the pre-acquired image with the ultrasonic image, and a display is used for displaying the registered pre-acquired image and ultrasonic image.

G. Diakov et al. "Automatische Registration des Patienten mit A-mode-Ultraschall fur computerunterstutzte Chirurgie", HNO 11 2010, DOI: 10.1007/s00106-010-2171-1 discloses a system in which the patient's head is supported in a stainless steel basin. An ultrasonic probe is positioned below the Teflon ^{®} floor in the x-direction and y-direction by means of stepper motors driving linear modules. The transmitter-receiver ultrasonic transducer and the module for position control are connected to the computer. The raw data of the measurement are transmitted via the network to a second computer where the surface of the occiputs is generated and the registration is carried out by means of iterative closest point (ICP).

US Patent application publication 2002/0035321 discloses a system in which an ultrasound probe is equipped with at least three emitters. The probe is placed over the body element of interest. The contour (which can be either two- or three-dimensional) of the body element is than obtained using the ultrasound probe. This contour can be expressed directly or indirectly in the procedural coordinates defined by the reference system (surgical space). Emitters communicate with sensors of reference array to indicate the position of the ultrasound probe. An ultrasound scanner which energizes probe determines the contour of the body element of interest and being scanned. This contour information is provided to processor for storage in intra-procedural geometry data memory. The intra-procedural contour stored in memory is then compared by a contour matching algorithm to a corresponding contour extracted from the pre-operative image data set stored in memory. Alternatively, a pre-procedural contour data set may be stored in memory based on a pre-procedural ultrasound scan which is input into memory via scanner interface prior to the procedure. This comparison process continues until a match is found for each one of the elements. Through this contour matching process, a registration is obtained between the images of each body element and the corresponding position of each element in the procedural space, thereby allowing the formation of the displaced image data set used for localization and display. Note that the contours used in the matching process only have to be sufficiently identical to accomplish a precise match-the contours do not have to be the same extent of the body element.

US6006126 provides a system for computer graphic determination and display of a patient's anatomy, from CT or MR scanning, along with associated equipment in an object field including the patient's anatomy. A first digitizing camera structure produces a signal representative of its field-of-view which defines coordinates of index points in its field-of-view. A second digitizing camera structure produces similar output for an offset field-of-view.

### SUMMARY OF THE INVENTION

The present invention is defined in the independent claim 1. Embodiments are defined in the dependent claims.

The present invention will be more fully understood from the following detailed description of the embodiments thereof, taken together with the drawings in which:

### BRIEF DESCRIPTION OF THE DRAWINGS

FIG. 1 is a schematic, pictorial illustration of a sinuplasty surgical system, in accordance with an embodiment of the present invention;
Fig. 2 is a schematic, pictorial illustration of a registration tool, in accordance with an embodiment of the present invention; and
Fig. 3 is a flow chart that schematically illustrates a method for registering an anatomical image with a coordinate system of a position tracking system.

### DETAILED DESCRIPTION OF EMBODIMENTS

### OVERVIEW

Some medical procedures such as sinuplasty require registration of an anatomical image of relevant organs with a coordinate system of a position tracking system. Using the registration, a surgical tool fitted with a position sensor is navigated to the treated organs, and is visualized overlaid on the anatomical image.

In principle, the registration may be carried out using some external registration tool fitted with a position sensor of the position tracking system. Such a tool could be attached to preselected locations on the patient face (e.g., nose tip, and centers of the two cheeks). The anatomical image could then be registered to the coordinate system of the position tracking system based on the measured positions of bone tissue at the preselected locations.

This possible solution, however, is likely to be inaccurate and unsuitable for sinuplasty procedures, in which it is typically important to obtain registration of the anatomical image at accuracy level better than 1 mm. Since facial elements comprise soft tissue that deforms naturally (e.g., due to changes in liquid level in the cheeks along the day), and because of the uncontrolled pressure applied by the registration tool thereon, the accuracy of this hypothetical solution may become unacceptable.

Embodiments of the present invention that are described hereinbelow provide improved techniques for registering an anatomical image with the coordinate system of a position-tracking system. In the disclosed embodiments, a registration tool comprises an ultrasound (US) transducer coupled to a position sensor of the position-tracking system. In order to perform registration, an operator (e.g., physician) attaches the registration tool to multiple predefined locations on the patient's face. At each of the predefined locations, the following measurements are performed:
▪ The position tracking system measures the position and orientation of the position sensor fitted in the registration tool.
▪ The US transducer images the bone tissue at the respective location.

A processor then uses the above measurements to calculate, for each of the predefined locations on the patient's face, the position of the respective bone tissue in the coordinate system of the position tracking system.

For a given predefined location on the patient's face, the output of the US transducer is indicative of the distance between the US transducer and the bone tissue. The relative displacement (if any) between the US transducer and the position sensor is fixed and known. The position of the position sensor has been measured in the coordinate system of the position tracking system. Therefore, the processor uses the above measurements to calculate the exact position of the bone tissue in the coordinate system of the position tracking system. This position is referred to herein as the "US coordinate" of the bone tissue for the predefined location on the patient face. The above procedure is repeated for each of the multiple predefined locations, to produce a set of US coordinates.

In addition, the processor identifies the positions of the bone tissue at the predefined multiple locations in a pre-acquired computerized tomography (CT) image. These positions are referred to herein as "CT coordinates" of the bone tissue. The processor then registers the CT image with the coordinate system of the position tracking system, e.g., by calculating a geometrical transformation that matches the US coordinates with the respective CT coordinates.

Since the disclosed registration process is based on correlation between coordinates of bone tissue, as opposed to soft tissue, it is highly accurate and insensitive to the impairments described above. The proposed techniques thus enable, for example, improved navigation of a sinuplasty surgical tool, which is inserted into the patient head and comprises another position sensor of the position-tracking system. Furthermore, the disclosed techniques are not sensitive to tissue deformation due to natural variations and due to the pressure applied by the registration tool.

### SYSTEM DESCRIPTION

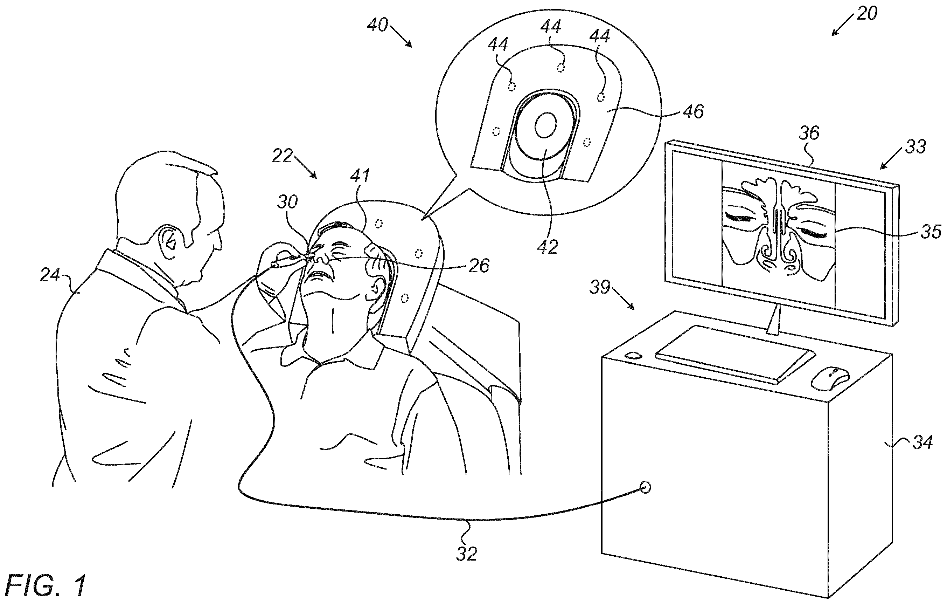

FIG. 1 is a schematic pictorial illustration of a sinuplasty surgical system 20, in accordance with an embodiment of the present invention. System 20 comprises a magnetic position tracking system, which is configured to track the position of one or more position sensors in the head of a patient 22. The magnetic position tracking system comprises magnetic field-generators and one or more position sensors. The position sensors generate position signals in response to sensed external magnetic fields from the field generators, thereby enabling a processor 34 to map the position of each sensor in the coordinate system of the position tracking system as will be described below.

This method of position sensing is implemented in various medical applications, for example, in the CARTO^{™} system, produced by Biosense Webster Inc. (Diamond Bar, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In the present example, system 20 comprises a location pad 40, which comprises multiple field-generators 44 fixed on a frame 46. In the exemplary configuration shown in FIG. 1, pad 40 comprises five field-generators 44, but any other suitable number of generators 44 can be used. Pad 40 further comprises a pillow 42 placed under a head 41 of patient 22, such that generators 44 are located at fixed, known positions external to the patient. System 20 further comprises a console 33, which comprises a driver circuit (not shown) configured to drive field-generators 44 with suitable signals so as to generate magnetic fields in a predefined working volume around head 41.

In an embodiment, processor 34 is typically a general-purpose computer comprising suitable front end and interface circuits for receiving data from external sources, as well as measurements from wand 30, via a cable 32, and for controlling other components of system 20. Console 33 further comprises input devices 39 and a user display 36, which is configured to display the data.

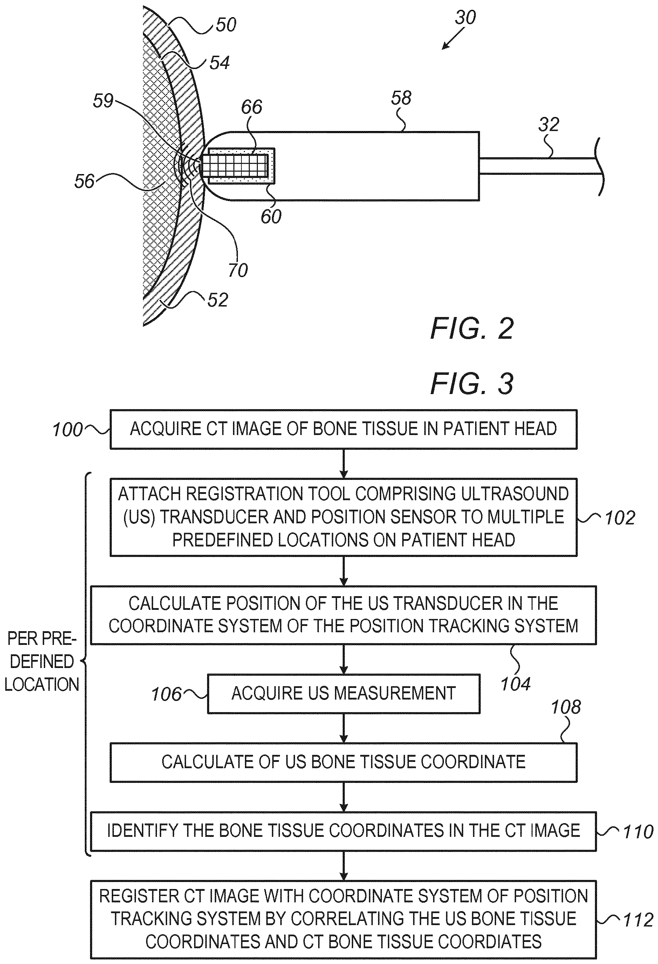

In some embodiments, system 20 comprises a registration tool, such as a handheld wand 30, which is used by system 20 for registering the coordinate system of the magnetic position tracking system with that of a pre-acquired CT image. The registration tool is configured to acquire ultrasound and position measurements, and is depicted in detail in Fig. 2 below.

Typically, a physician 24 attaches wand 30 sequentially to multiple predefined locations on an external surface of patient head 41. Each predefined location is typically chosen to be an easily identifiable feature on head 41, such as a cheek bone protrusion, a bridge of a nose 26 (located between the eyes of patient 22), a tip of nose 26, a chin, or any other suitable identifiable feature.

In an embodiment, processor 34 receives a computerized tomography (CT) image 35 obtained using an external CT system (not shown). Processor 34 uses image 35 to form a surface image of at least part of patient head 41. In some embodiments, processor 34 may use hounsfield units (HU) ranging between 700 and 3000 for determining the radiodensity of bones in the patient face, compared with HU of -1000, which is a standard scale for air, so as to determine boundaries of the patient face. In an alternative embodiment, HU above 500 may be used for determining the radiodensity of bones, HU of -200 and below may be used for air, and HU ranging between -200 and 500 may be used for muscular tissue. Alternatively, any other suitable values can be used. Further alternatively, processor 34 may distinguish between different types of tissue in the CT image, and in particular identify bone tissue, using any other suitable criterion or technique.

In an embodiment, when placed at a predefined location on the patient head, wand 30 is configured to (i) acquire US measurements of bone tissue, and (ii) generate position signals indicative of the position of this predefined location in the coordinate system of the magnetic position tracking system. The acquisition of the bone tissue measurements by wand 30 is described in detail in Fig. 2 below.

In some embodiments, processor 34 is configured to calculate two coordinates for each predefined location on the patient head - A "US coordinate" and a "CT coordinate." The US coordinate is derived from the US and position measurements of wand 30 at this predefined location, and is indicative of the coordinate of the bone tissue at this location in the coordinate system of the magnetic position tracking system. The CT measurement is indicative of the coordinate of the bone tissue at this location, as identified in the CT image.

In an embodiment, processor 34 is configured to correlate between the US coordinates and the CT coordinates of the predefined locations in image 35, so as to register the CT image with the coordinate system of the position tracking system.

The registration process is typically performed before the actual sinuplasty procedure. During the sinuplasty procedure, physician 24 may insert into head 41 a medical device (not shown), such as a sinuplasty catheter or other surgical tool, which comprises an additional position sensor of the position-tracking system. Since the CT image is already registered with the position-tracking system, physician 24 may navigate the medical device whose distal end is displayed on the CT image, to a target location in head 41.

In alternative embodiments, instead of CT image 35, processor 34 is configured to receive one or more images acquired using another suitable anatomical imaging technique, such as fluoroscopy or magnetic resonance imaging (MRI), and to register these anatomical images with the coordinate system as described above.

Fig. 1 shows only elements related to the disclosed techniques, for the sake of simplicity and clarity. System 20 typically comprises additional modules and elements that are not directly related to the disclosed techniques, and thus, intentionally omitted from Fig. 1 and from the corresponding description.

Processor 34 may be programmed in software to carry out the functions that are used by the system, and to store data in a memory (not shown) to be processed or otherwise used by the software. The software may be downloaded to the processor in electronic form, over a network, for example, or it may be provided on non-transitory tangible media, such as optical, magnetic or electronic memory media. Alternatively, some or all of the functions of processor 34 may be carried out by dedicated or programmable digital hardware components.

### REGISTERING ANATOMICAL IMAGES WITH A POSITION-TRACKING SYSTEM USING ULTRASOUND

Fig. 2 is a schematic, pictorial illustration of wand 30, in accordance with an embodiment of the present invention. The figure shows wand 30 placed at one of the multiple predefined locations on patient head 41. In some embodiments, wand 30 comprises a housing 58, which contains a position sensor 60 concentrically disposed around an ultrasound (US) transducer 66. The US transducer is configured to produce US pulses 70 into the tissue. Fig. 2 shows example tissue structure, which comprises a skin layer 50, an intermediate tissue 52, a bone tissue 56 and an interface layer 54 between tissue 52 and 56.

In an embodiment, physician 24 attaches a tip 59 of wand 30 to skin 50 at one of the predefined locations (e.g., bridge of nose 26), and activates transducer 66 so as to produce US pulses 70. US Pulses 70 traverse skin layer 50 and intermediate tissue 52 toward interface layer 54 and bone tissue 56. Pulses 70 are reflected from interface 54 and travel through tissue 52 and skin layer 50, back to wand 30.

Wand 30 is configured to measure round-trip propagation times of the US pulses, also denoted time-of-flight (TOF). Based on the known speed of the US pulses in tissue 52 and skin layer 50, processor 34 is configured to translate the measured TOF into a thickness of tissue 52, i.e., into the distance between transducer 66 and bone tissue 56. In an embodiment, wand 30 is configured to transmit the position measurements obtained from sensor 60, and the TOF measured using US transducer 66, via cable 32, to processor 34.

Sensor 60 is concentrically disposed around transducer 66. Using the position of sensor 60 and the TOF measurement of transducer 66, processor 34 is configured to calculate the US coordinate, i.e., the position of the bone tissue in the coordinate system of the position tracking system.

In an embodiment sensor 60 may be fitted at a fixed, known displacement relative to transducer 66, thus, processor 34 may take into account the fixed displacement in calculating the US coordinate of the bone tissue.

As depicted in Fig. 2, the pressure applied by tip 59 may deform skin layer 50 and tissue 52. Processor 34 essentially prevents such deformation from distorting the registration, since the registration is based on bone tissue correlation.

Fig. 3 is a flow chart that schematically illustrates a method for registering CT image 35 with the coordinate system of the position tracking system. The method begins with a CT image acquisition step 100, in which processor 34 receives one or more CT images that capture bone tissue (referred to herein as CT bone images) of head 41.

At a registration tool attachment step 102, physician 24 attaches wand 30 sequentially to the multiple predefined locations in head 41. The subsequent steps (104-110) are performed at each of these predefined locations.

At a position calculation step 104, processor 34 receives the position measurement from sensor 60 and calculates the position of transducer 66 in the coordinate system of the position tracking system. At an US measurements step 106, system 20 activates transducer 66 so as to acquire US measurements. At a tissue position calculation step 108, processor 34 receives the position signals from transducer 66 and the TOF measurement from transducer 66, and calculates the US coordinate, i.e., the position of the bone tissue in the coordinate system of the position tracking system. At a CT tissue identification step 110, processor 34 identifies the corresponding CT coordinate, i.e., the position of the bone tissue in CT image 35, using any suitable technique known in the art.

As noted above, steps 104-110 are repeated per each predefined location.

At a registration step 112, processor 34 registers CT image 35 with the coordinate system of the position tracking system by correlating between the US bone tissue coordinates and the corresponding CT bone tissue coordinates at the predefined locations in head 41.

Although the embodiments described herein mainly address sinuplasty procedures, the methods and systems described herein can also be used in other applications, such as in orthopedic procedures, in which physician 24 may attach wand 30 to any suitable deformable feature on a human body.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which fall within the scope of the appended claims.

## Claims

1. An apparatus, comprising:
a handheld registration tool (30), comprising:
an ultrasound (US) transducer (66), which is configured, when the registration tool is attached sequentially to multiple respective predefined locations on a patient head (41), to acquire respective US measurements of bone tissue at the predefined locations; and
a position sensor (60) of a position-tracking system, which is configured to acquire respective position measurements of the registration tool at the predefined locations, wherein the position sensor (60) is coupled to the US transducer (66) and the position sensor (60) is concentrically disposed around the US transducer (66); and
a processor (34), which is configured to:
receive the multiple US measurements and the respective position measurements acquired by the registration tool;
calculate first positions of the bone tissue at the multiple predefined locations, based on the position measurements and the US measurements;
identify second positions of the bone tissue at the multiple predefined locations, in an anatomical image of the patient head; and
register the anatomical image with a coordinate system of the position-tracking system, by correlating the first positions and the second positions, so as to enable tracking a medical instrument, which is inserted into the patient head and comprises another position sensor of the position-tracking system, using the anatomical image registered with the position-tracking system.

2. The apparatus according to claim 1, wherein the anatomical image comprises one or more computerized tomography (CT) images.

3. The apparatus according to claim 1, wherein the predefined locations comprise locations of bone features selected from a list consisting of a cheek bone protrusion, a bridge of a nose (26), a tip of the nose and a chin.

4. The apparatus according to claim 1, wherein the position-tracking system comprises a magnetic position-tracking system.

5. The apparatus according to claim 1, wherein the multiple US measurements comprise round-trip propagation times of US pulses traversing at each location between an external surface of the head and the respective bone tissue.

6. The apparatus according to claim 1, wherein the medical instrument comprises a sinuplasty catheter.

## Patentansprüche

1. Vorrichtung, umfassend:
ein handgeführtes Registrierungswerkzeug (30), umfassend:
einen Ultraschall (US)-Wandler (66), der, wenn das Registrierungswerkzeug sequenziell an mehreren entsprechenden vordefinierten Stellen an einem Kopf des Patienten (41) angebracht wird, dazu ausgelegt ist, entsprechende US-Messungen von Knochengewebe an den vordefinierten Stellen zu erfassen; und
einen Positionssensor (60) eines Positionsverfolgungssystems, der dazu ausgelegt ist, entsprechende Positionsmessungen des Registrierungswerkzeugs an den vordefinierten Stellen zu erfassen, wobei der Positionssensor (60) mit dem US-Wandler (66) gekoppelt ist, und der Positionssensor (60) konzentrisch um den US-Wandler (66) angeordnet ist; und
einen Prozessor (34), der dazu ausgelegt ist:
die mehreren US-Messungen und die von dem Registrierungswerkzeug erfassten entsprechenden Positionsmessungen zu empfangen;
erste Positionen des Knochengewebes an den mehreren vordefinierten Stellen zu berechnen, basierend auf den Positionsmessungen und den US-Messungen;
zweite Positionen des Knochengewebes an den mehreren vordefinierten Stellen zu identifizieren, in einem anatomischen Bild des Patientenkopfes; und
das anatomische Bild in einem Koordinatensystem des Positionsverfolgungssystems zu registrieren, durch Korrelieren der ersten Positionen und zweiten Positionen,
um das Verfolgen eines medizinischen Instruments zu ermöglichen, das in den Patientenkopf eingesetzt wird und einen anderen Positionssensor des Positionsverfolgungssystems umfasst, mithilfe des anatomischen Bildes, das mit dem Positionsverfolgungssystem registriert ist.

2. Vorrichtung nach Anspruch 1, wobei das anatomische Bild ein oder mehrere Computertomografie (CT)-Bilder umfasst.

3. Vorrichtung nach Anspruch 1, wobei die vordefinierten Stellen Stellen von Knochenmerkmalen umfassen, die aus einer Liste bestehend aus einer Wangenknochenwölbung, einem Nasenrücken (26), einer Nasenspitze und einem Kinn ausgewählt sind.

4. Vorrichtung nach Anspruch 1, wobei das Positionsverfolgungssystem ein magnetisches Positionsverfolgungssystem umfasst.

5. Vorrichtung nach Anspruch 1, wobei die mehreren US-Messungen Umlaufzeiten von US-Impulsen umfassen, die an jeder Stelle zwischen einer Außenseite des Kopfes und dem entsprechenden Knochengewebe durchlaufen.

6. Vorrichtung nach Anspruch 1, wobei das medizinische Instrument einen Sinuplastiekatheter umfasst.

## Revendications

1. Appareil comprenant :
un outil d'enregistrement portatif (30), comprenant :
un transducteur à ultrasons (US) (66), qui est configuré, lorsque l'outil d'enregistrement est fixé séquentiellement à de multiples emplacements prédéfinis respectifs sur la tête d'un patient (41), pour acquérir des mesures US respectives de tissu osseux aux emplacements prédéfinis ; et
un capteur de position (60) d'un système de suivi de position, qui est configuré pour acquérir des mesures de position respectives de l'outil d'enregistrement aux emplacements prédéfinis, le capteur de position (60) étant couplé au transducteur US (66) et le capteur de position (60) étant disposé concentriquement autour du transducteur US (66) ; et
un ordinateur (34) qui est configuré pour :
recevoir les multiples mesures US et les mesures de position respectives acquises par l'outil d'enregistrement ;
calculer des premières positions du tissu osseux aux multiples emplacements prédéfinis, sur la base des mesures de position et des mesures US ;
identifier des secondes positions du tissu osseux aux multiples emplacements prédéfinis, dans une image anatomique de la tête du patient ; et
enregistrer l'image anatomique avec un système de coordonnées du système de suivi de position, en corrélant les premières positions et les secondes positions, afin de permettre le suivi d'un instrument médical qui est inséré dans la tête du patient et comprend un autre capteur de position du système de suivi de position, à l'aide de l'image anatomique enregistrée avec le système de suivi de position.

2. Appareil selon la revendication 1, l'image anatomique comprenant une ou plusieurs images de tomodensitométrie (CT).

3. Appareil selon la revendication 1, les emplacements prédéfinis comprenant des emplacements d'éléments osseux choisis dans une liste comprenant une protubérance de l'os de la joue, une arête du nez (26), une pointe du nez et un menton.

4. Appareil selon la revendication 1, le système de suivi de position comprenant un système de suivi de position magnétique.

5. Appareil selon la revendication 1, les multiples mesures US comprenant des temps de propagation aller-retour d'impulsions US traversant à chaque emplacement entre une surface externe de la tête et le tissu osseux respectif.

6. Appareil selon la revendication 1, l'instrument médical comprenant un cathéter de sinuplastie.
